# EUROPEAN PATENT SPECIFICATION

(11) **EP 2 421 914 B1**
(45) Date of publication and mention of the grant of the patent: **30.03.2016**
(21) Application number: 10716014.5
(22) Date of filing: 16.04.2010
(51) Int. Cl.: C08K 3/30, A61L 27/18, A61L 27/44

(54) **POLYMERIC MATERIALS COMPRISING BARIUM SULPHATE**
POLYMERMATERIALIEN MIT BARIUMSULFAT
MATÉRIAUX POLYMÈRES COMPRENANT DU SULFATE DE BARYUM

(30) Priority: 21.04.2009 GB 0906823
(43) Date of publication of application: 29.02.2012
(73) Proprietor: Invibio Limited, Lancashire FY5 4QD (GB)
(72) Inventor: VALENTINE, Craig, Lancashire FY5 4QD (GB); ELLERAY, Andrew, Lancashire BB5 2PF (GB); CARTWRIGHT, Keith, Lancashire FY5 4QD (GB)
(74) Representative: Marsh, Andrew James
(86) International application number: PCT/GB2010/050631
(87) International publication number: WO 2010/122326

(56) References cited:
- EP-A1- 0 423 510
- EP-A1- 0 451 458
- GB-A- 2 435 648
- JP-A- 2005 171 242
- US-A1- 2008 234 532
- Invibio© biomaterial solutions: "PEEK-OPTIMA POLYMER Performance Purity Flexibility Endurance" 2004, XP002588809 Retrieved from the Internet: URL:http://www.ppchase.com/cayenne/downloa d/cay14.pdf [retrieved on 2010-06-24]

## Description

This invention relates to polymeric materials and particularly, although not exclusively, relates to polymeric materials with improved Notched Izod Impact Strength.

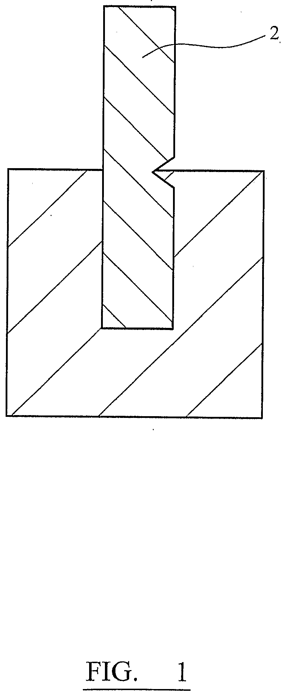

Notched Izod Impact Strength (NIIS) may be assessed in accordance with ISO180 wherein a specimen 2 as shown in figure 1 is held as a vertical cantilevered beam and is broken by a pendulum. Impact occurs on the notched side of the specimen.

The NIIS test may be of use in assessing the strength of parts made from polymeric materials which have a notch (or a shape similar to a notch) as part of the design or may be of use in assessing the impact on the strength of a part by damage which may produce a notch (or a shape similar to a notch) in the part.

In the field of orthopaedic implants, the notch sensitivity is, in some cases, a potential cause of failure of an implant. For example, dynamic stabilization rods may be pre-notched during manufacture or may be notched during the surgical procedure. The notched areas have been identified as possible failure points. Similarly, in hip joints, there is potential for acetabular cup impingement during implantation which could result in notching and the risk of fracture. In general terms, notch sensitivity may be important in sports medicine applications (e.g. for suture anchors and screws), in trauma (e.g. for plates, nails and screws) and in orthopaedics (e.g. for acetabular cups, femoral heads and humeral heads).

In many non-medical applications NIIS is important, particularly if there is a risk that a part may become damaged so that a notch is defined.

It is an object of the present invention to address the above described problems.

According to a first aspect of the invention, there is provided the use according to claim 1.

Said polymeric material may be amorphous or semi-crystalline. Said polymeric material is preferably semi-crystalline. The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calerimetry (DSC).

The level of crystallinity in said polymeric material may be at least 1%, suitably at least 3%, preferably at least 5% and more preferably at least 10%. In especially preferred embodiments, the crystallinity may be greater than 30%, more preferably greater than 40%, especially greater than 45%.

The main peak of the melting endotherm (Tm) for said polymeric material (if crystalline) may be at least 300°C.

Said polymeric material preferably consists essentially of a repeat unit of formula (XX) 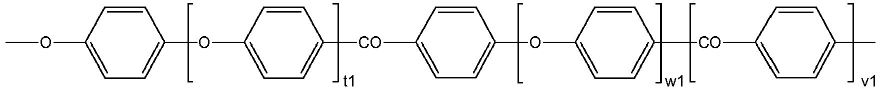 where t1, and w1 independently represent 0 or 1 and v1 represents 0, 1 or 2. Preferred polymeric materials of formula (XX) have a said repeat unit wherein t1=1, v1=0 and w1=0; t1=0, v1=0 and w1=0; t1=0, w1=1, v1=2; or t1=0, v1=1 and w1=0. More preferred have t1=1, v1=0 and w1=0; or t1=0, v1=0 and w1=0. The most preferred has t1=1, v1=0 and w1=0.

In preferred embodiments, said polymeric material is selected from polyetheretherketone, polyetherketone, polyetherketoneetherketoneketone and polyetherketoneketone. In a more preferred embodiment, said polymeric material is selected from polyetherketone and polyetheretherketone. In an especially preferred embodiment, said polymeric material is polyetheretherketone.

Said polymeric material may have a Notched Izod Impact Strength (specimen 80mm x 10mm x 4mm with a cut 0.25mm notch (Type A), tested at 23°C, in accordance with ISO180) (hereinafter referred to as NIIS) of at least 4KJm⁻², preferably at least 5KJm⁻², more preferably at least 6KJm⁻². Said Notched Izod Impact Strength, measured as aforesaid, may be less than 10KJm⁻², suitably less than 8KJm⁻².

NIIS hereinafter referred to is measured as aforesaid, unless otherwise stated.

Said polymeric material suitably has a melt viscosity (MV) of at least 0.06 kNsm⁻², preferably has a MV of at least 0.085 kNsm⁻², more preferably at least 0.12 kNsm⁻², especially at least 0.14 kNsm⁻².

MV is suitably measured using capillary rheometry operating at 400°C at a shear rate of 1000s⁻¹ using a tungsten carbide die, 0.5x3.175mm.

Said polymeric material may have a MV of less than 1.00 kNsm⁻², preferably less than 0.5 knsm⁻².

Said polymeric material may have a MV in the range 0.09 to 0.5 kNsm⁻², preferably in the range 0.14 to 0.5 kNsm⁻².

Said polymeric material may have a tensile strength, measured in accordance with ISO527 (specimen type 1 b) tested at 23°C at a rate of 50mm/minute of at least 20 MPa, preferably at least 60 MPa, more preferably at least 80 MPa. The tensile strength is preferably in the range 80-110 MPa, more preferably in the range 80-100 MPa.

Said polymeric material may have a flexural strength, measured in accordance with ISO178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa.

Said polymeric material may have a flexural modulus, measured in accordance with ISO178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

For the avoidance of doubt, the aforesaid characteristics of said polymeric material refer to polymeric material per se (ie unfilled).

Said polymeric material may be amorphous or semi-crystalline. It is preferably semi-crystalline.

The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calerimetry (DSC).

The level of crystallinity of said polymeric material may be at least 1%, suitably at least 3%, preferably at least 5% and more preferably at least 10%. In especially preferred embodiments, the crystallinity may be greater than 25%.

The main peak of the melting endotherm (Tm) of said polymeric material (if crystalline) may be at least 300°C.

Said barium sulphate may have a D₁₀ particle size in the range 0.1 to 1.0µm; a D₅₀ particle size suitably in the range 0.5µm to 2µm and a D₉₀ particle size suitably in the range 1.0µm to 5µm. The D₁₀ may be in the range 0.1 to 0.6µm, preferably 0.2 to 0.5µm. The D₅₀ may be in the range 0.7µm to 1.5µm, preferably 0.8 to 1.3µm. The D₉₀ may be in the range 1.5 to 3µm, preferably in the range 2.0 to 2.5µm.

The ratio of the wt% of barium sulphate to the wt% of said polymeric material is suitably greater than 0.07, is preferably greater than 0.10, is more preferably greater than 0.13, and is especially greater than 0.16. In some cases, said ratio may be greater than 0.20 or 0.22. The ratio may be less than 0.4, or less than 0.3.

Said barium sulphate is preferably intimately mixed with the polymeric material, suitably so the barium sulphate and polymeric material define a substantially homogenous mixture.

The wt% of barium sulphate in a composition which includes barium sulphate and said polymeric material may be at least 5wt%, suitably at least 8wt%, preferably at least 10wt%, more preferably at least 13wt%, especially at least 16wt%. Said wt% of barium sulphate may be less than 30wt% or less than 25wt%. The wt% of said polymeric material in a composition which includes barium sulphate (and, optionally water as hereinafter described) may be at least 40wt%, preferably at least 45wt%, more preferably at least 50wt%. In some cases, greater than 99wt% of said composition may consist essentially of barium sulphate and said polymeric material. In other cases, said composition may include one or more other fillers, for example carbon fibre. A composition may include up to 35wt% or up to 30wt% carbon fibre. When carbon fibre is included the composition suitably includes 25-35wt% carbon fibre.

Said barium sulphate may be used to increase the NIIS of said polymeric material by at least 10 KJm⁻², suitably by at least 20KJm⁻², preferably by at least 30 KJm⁻², more preferably by at least 40 KJm⁻², measured in accordance with ISO180. Said NIIS may be increased by less than 60KJm⁻².

The ratio of the NIIS of a composition which includes barium sulphate as described to the NIIS of an otherwise identical composition which does not include barium sulphate as described may be at least 2, suitably at least 4, preferably at least 6, more preferably at least 8. The ratio may be less than 15 or less than 11.

The NIIS of a composition comprising said polymeric material and barium sulphate is increased when the composition includes water.

The ratio of the wt% of water to barium sulphate may be in the range 0.01 to 0.2, preferably in the range 0.02 to 0.15. The ratio is suitably less than 0.10, preferably less than 0.05, more preferably less than 0.04.

The wt% of water in a composition which also includes barium sulphate and said polymeric material may be at least 0.10wt%, preferably at least 0.2wt%, more preferably at least 0.30wt%, especially at least 0.40wt%. The wt% of water may be less than 1wt%, less than 0.8wt%, less than 0.6wt%. The wt% of water may be in the range 0.3 to 0.7wt%, suitably 0.4 to 0.6wt%.

Said composition comprising polymeric material and barium sulphate may be provided in any desired form, for example as a stock shape, film or fibre.

The NIIS of said composition comprising polymeric material and barium sulphate has been found to be reversible in dependence upon whether water is present or not. To benefit from the increase in impact strength it is therefore desirable for a part made from a composition comprising barium sulphate and said polymeric material to be used in a moist and/or wet environment. Thus, the invention extends to the use of barium sulphate for increasing the impact strength of a polymeric material according to the first aspect used in making a part for use in a moist and/or wet environment. Said environment may include water in a liquid state. Water in a liquid state may contact the part in use. Said environment may be substantially enclosed and suitably includes a source of water and/or water vapour. Said environment may comprise a human or animal body (especially the former) and preferably comprises an internal region of a human body. Said part may be an implant.

According to a second aspect of the invention, there is provided a method of increasing the impact strength of a polymeric material as in claim 8.

Said composition, polymeric material, barium sulphate, impact strength and any other features of the invention of the first aspect may be applied to the invention of the second aspect mutatis mutandis.

The method may comprise selecting a said polymeric material and melt compounding it with barium sulphate.

The method may comprise treating the composition to increase the level of water contained therewithin.

In a preferred embodiment, there is provided a method of increasing the impact strength of a part which comprises a polymeric material as described according to the first aspect and barium sulphate, the method comprising treating the part to increase the level of water contained therewithin.

The part may have any feature of the composition described according to the first and second aspects after said treatment.

The wt% of water in said part which also includes barium sulphate and said polymeric material may be at least 0.10wt%, preferably at least 0.20wt%, more preferably at least 0.30wt%, especially at least 0.40wt%. The wt% of water may be less than 1wt%, less than 0.8wt%, less than 0.6wt%. The wt% of water may be in the range 0.3 to 0.7wt%, suitably 0.4 to 0.6wt%.

The wt% of barium sulphate in said part may be at least 5wt%, suitably at least 8wt%, preferably at least 10wt%, more preferably at least 13wt%, especially at least 16wt%. Said wt% of barium sulphate may be less than 30wt% or less than 25wt%.

The wt% of said polymeric material in said part may be at least 40wt%. In some cases, greater than 99wt% of said part may consist essentially of barium sulphate and said polymeric material. In other cases, said part may include one or more other fillers, for example carbon fibre. A part may include up to 35wt% or up to 30wt% carbon fibre. When carbon fibre is included the part suitably includes 25-35wt% carbon fibre.

Treatment of said part preferably involves an active treatment. It preferably comprises a treatment at a temperature of greater than 40°C, suitably at greater than 60°C, preferably at greater than 80°C. The part may be treated with steam. The part may be treated under a pressure of greater than ambient pressure, for example greater than 2 bar or 4 bar. Treatment may involve an autoclave and/or may involve steam sterilisation.

The invention extends to a part as described in claim 10.

Preferably said part has been treated to increase its NIIS as described in a method of the second aspect.

The part is preferably spaced from and/or separated from its position of intended use. The part is preferably outside an environment in which it is intended to be used. For example, if the part is for implantation in a human or animal body (as is preferred) the part may be outside the body but suitably has increased impact strength by virtue of the treatment to which it has been subjected and/or other features of the part described herein.

Said part is preferably an implantable part, for example an implantable prosthesis, such as an orthopaedic implant, for implantation in a human body. It may be a dynamic stabilisation rod or for use in a prosthetic hip joint.

Said part may comprise greater than 50wt% of said polymeric material (preferably polyetheretherketone), at least 8wt% (preferably at least 10wt% or at least 13wt%) barium sulphate and at least 0.3wt% (preferably at least 0.45wt%) of water. In an especially preferred embodiment, said part includes at least 55wt% polyetheretherketone, at least 10wt% barium sulphate and at least 0.4wt% of water. The part may include 0 to 34.6wt% of other fillers, for example carbon fibre.

In a preferred embodiment, there is provided a package, which is suitably sterile, comprising a part as described.

Said part may be arranged in a package from which air has been removed. For example, it may be vacuum packed for example in a metal (e.g. aluminium) lined receptacle.

In a preferred embodiment, there is provided the use of a composition comprising a polymeric material according to the first aspect and barium sulphate in the manufacture of an implantable prosthesis for example an orthopaedic implant.

In a preferred embodiment, there is provided a method of making an implantable prosthesis, for example an orthopaedic implant, the method comprising:
(i) selecting a composition comprising a said polymeric material according to the first aspect and barium sulphate;
(ii) melt processing, for example extruding or injection moulding, said composition to define the part or a precursor of said part.

The impact strength of the part may be increased as described according to the second aspect.

Any feature of any aspect of any invention or embodiment described herein may be combined with any feature of any aspect of any other invention or embodiment described herein mutatis mutandis.

Specific embodiments of the invention will now be described by way of example.

The following materials are referred to hereinafter.

PEEK-OPTIMA LT1 (Trade Mark) - an implantable grade of polyetheretherketone having an MV of 0.46 KNsm⁻² obtained from Invibio Limited, Thornton, Cleveleys, UK.

Barium Sulphate - Grade 101750, extra pure for X-ray diagnosis.

The influence of barium sulphate on properties of polyetheretherketone was investigated in a range of experiments as described in the following examples.

### Example 1 - General procedure for preparing polvetheretherketone/barium sulphate composites

Barium sulphate was added to PEEK-OPTIMA via an extrusion compounding process. By way of example, the barium sulphate can be gravimetrically metered and fed through a side feeder into a twin screw extruder, where it is combined with plasticized polymer melt and intimately mixed to provide a uniform dispersion of the filler within the polymer. Typically the filler may be added up to 60% by weight of the polymer, depending upon the desired mechanical properties of the compound. Extrusion of this mixture through a die generates strands or laces that cool and solidify before being chopped into small granules in preparation for subsequent processing.

### Example 2 - General procedure for preparing test pieces of composites

The compound prepared in Example 1 was injection moulded to manufacture test pieces, in accordance with the requirements of ISO 527-2 (sample geometry 1B), ISO 178, and ISO 180 for tensile strength, flexural strength and notched Izod impact testing respectively.

### Examples 3 - 20 - Preparation and testing of test pieces

Test pieces were prepared as described in Examples 1 and 2 containing PEEK-OPTIMA LT1 and either 4wt% or 20wt% of barium sulphate and subjected to a range of different treatments as described in Table 1.

Each test piece was tested in accordance with ISO180 for notched ISOD impact strength. ISO572-2, ISO178 and for tensile properties, flexural properties and ISO180 for notched IZOD impact strength. Results are provided in Table 2.

**Table 1**

| **Pre-Treatment** | **Amount of Barium Sulphate** | |
|---|---|---|
| | **4%wt** | **20wt%** |
| A) As moulded | Example 3 | Example 12 |
| B) 60 day natural ageing (closed container) | Example 4 | Example 13 |
| C) 60 day natural ageing (open to atmosphere) | Example 5 | Example 14 |
| D) 60 day natural ageing plus 200kGy gamma | Example 6 | Example 15 |
| E) 60 day natural ageing plus 200kGy gamma plus oxygen ageing (40 days at 5 bar) | Example 7 | Example 16 |
| F) 60 day natural ageing plus 200kGy gamma plus oxygen ageing (40 days at 5 bar) plus saline soak (3 months at 90°C) to simulate 10 years in-vivo | Example 8 | Example 17 |
| G) 60 day natural ageing plus oxygen ageing (40 days at 5 bar) plus steam sterilisation (3 cycles) plus saline soak (3 months at 90°C) | Example 9 | Example 18 |
| H) 60 day natural ageing plus 3 steam cycles (134°C) | Example 10 | Example 19 |
| I) 60 days natural ageing plus 3 EtO cycles | Example 11 | Example 20 |

**Table 2**

| **Example No** | **Notched Izod Impact KJ/m²** |
|---|---|
| 3 | 7.97 |
| 4 | 7.2 |
| 5 | 10.71 |
| 6 | 11.73 |
| 7 | 7.86 |
| 8 | 11.63 |
| 9 | 9.01 |
| 10 | 9.7 |
| 11 | 10.58 |
| 12 | 7.7 |
| 13 | 6.13 |
| 14 | 19.15 |
| 15 | 20.36 |
| 16 | 16 |
| 17 | 48.03 |
| 18 | 26.34 |
| 19 | 53.99 |
| 20 | 20.76 |

### Examples 21 - 25 - Comparison of dried and "wet" test pieces

Test pieces were prepared as described in Example 2 with different filler loadings, varying between 4 to 20wt% and the Notched Izod Impact Strength assessed in accordance with ISO180 under two different sets of conditions - firstly, after drying in an oven at 120°C for 72 hours; and, secondly, after sterilisation in steam for 20 minutes at 134°C, soaking in water for 15 hours followed by further sterilisation in steam for 20 minutes at 134°C. Results are provided in Table 3.

**Table 3**

| **Example No** | **Amount of barium sulphate wt%** | **Notched Izod Impact KJ/m²** | |
|---|---|---|---|
| | | **After Drying** | **After sterilisation/soaking/sterilisation** |
| 21 | 4 | 5.08 | 5.6 |
| 22 | 6 | 4.36 | 6.75 |
| 23 | 10 | 3.82 | 7.58 |
| 24 | 15 | 3.74 | 8.6 |
| 25 | 20 | 4.17 | 16.54 |

### Example 26 - Cyclical Testing

Respective test pieces comprising 4wt% and 20wt% barium sulphate were prepared and tested in cyclical testing.

Firstly, a 4wt% barium sulphate test piece was tested after moulding; then it was dried at 120°C for 72 hours and re-tested; thereafter it was sterilised in steam for 20 minutes at 134°C and re-tested; and finally it was re-dried and re-tested. Results are provided in Table 4.

**Table 4**

| **Example No** | **Amount barium sulphate (wt%)** | **Time when test undertaken** | **Notched Izod Impact KJ/m²** |
|---|---|---|---|
| 26 | 4 | After moulding | 7.97 |
| 27 | 4 | After initial drying | 6.74 |
| 28 | 4 | After sterilization | 9.64 |
| 29 | 4 | After re-drying | 4.64 |
| 30 | 20 | After moulding | 7.7 |
| 31 | 20 | After initial drying | 5.37 |
| 32 | 20 | After sterilization | 29.8 |
| 33 | 20 | After re-drying | 5.79 |

### Discussion

The effect on impact strength of the incorporation of barium sulphate and water into polyaryletherketone may have many industrial applications. It may be of particular utility for making parts which are used in a moist environment so that the optimum amount of water (believed to be about 0.15wt%) may be maintained within a composition which comprises the polyaryletherketone and barium sulphate. A suitably moist environment is found in the human body and, consequently, parts made from polyaryletherketone and barium sulphate and, optionally, other fillers, may be pre-conditioned so they contain water, for example 0.15wt%, and then implanted. When implanted, the water level is maintained and so, therefore, is the surprising improvement in impact strength. It is also found that other properties, for example other mechanical properties of the polyaryletherketone are not significantly detrimentally affected by incorporation of barium sulphate and/or water. Furthermore the polyaryletherketone is chemically unchanged.

## Claims

1. The use of barium sulphate and water for increasing the Notched Izod Impact Strength (determined in accordance with ISO180 as described in the description) of a polymeric material which comprises a repeat unit of formula 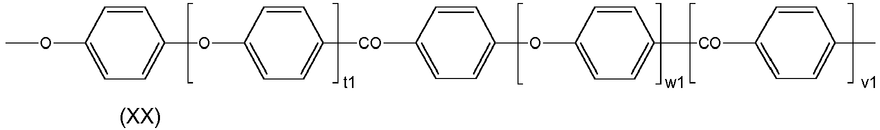 where t1 and w1 independently represent 0 or 1 and v1 represents 0, 1 or 2, wherein the ratio of the wt% of barium sulphate to the wt% of polymeric material is greater than 0.04 and is less than 0.4; wherein the wt% of said polymeric material in a composition which includes said barium sulphate is at least 50wt%.

2. The use according to claim 1, wherein c1=1, v1=0 and w1=0.

3. The use according to claim 1 or claim 2, wherein the ratio of the wt% of barium sulphate to the wt% of said polymeric material is less than 0.3.

4. The use according to any preceding claim, wherein the wt% of barium sulphate in a composition which includes barium sulphate and said polymeric material is at least 10wt%.

5. The use according to any preceding claim, wherein said barium sulphate is used to increase the Notched Izod Impact Strength of said polymeric material by at least 10KJm⁻².

6. The use according to any preceding claim, wherein the ratio of the wt% of water to barium sulphate is in the range 0.01 to 0.2.

7. The use according to any preceding claim, wherein the wt% of water in a composition which includes barium sulphate and said polymeric material is less than 1wt%.

8. A method of increasing the Notched Izod Impact Strength (determined in accordance with ISO180 as described in the description) of a polymeric material which comprises a repeat unit of formula 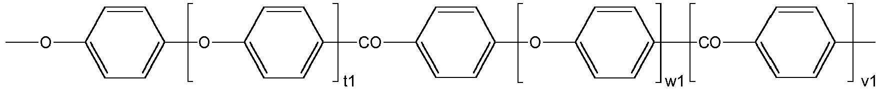 where t1 and w1 independently represent 0 or 1 and v1 represents 0, 1 or 2, the method comprising forming a composition comprising said polymeric material and barium sulphate, wherein the ratio of the wt% of barium sulphate to the wt% of polymeric material is greater than 0.04 and is less than 0.4; and wherein the wt% of said polymeric material in a composition which includes said barium sulphate is at least 50wt%, wherein the method comprises an active treatment of said composition to increase the level of water contained therewithin.

9. A method according to claim 8, wherein the wt% of water in said part which includes barium sulphate and said polymeric material is at least 0.30wt% and is less than 1wt%; the wt% of barium sulphate in said part is at least 10wt% and is less than 30wt%.

10. A part which comprises a polymeric material which comprises a repeat unit of formula 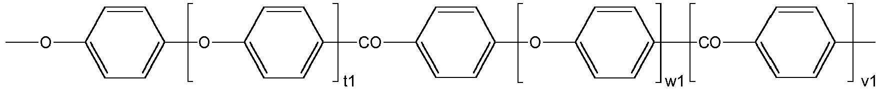 where t1 and w1 independently represent 0 or 1 and v1 represents 0, 1 or 2, wherein the ratio of the wt% of barium sulphate to the wt% of polymeric material is greater than 0.04 and is less than 0.4; wherein the wt% of said polymeric material in a composition which includes said barium sulphate is at least 50wt%; and wherein said part comprises at least 0.1wt% water, preferably at least 0.2wt%, more preferably at least 0.30wt% water.

11. A part according to claim 10, wherein said part is provided in a package.

12. A part according to claim 11, wherein said part is vacuum packed.

13. A part according to claim 10, said part being an implantable prosthesis.

## Patentansprüche

1. Verwendung von Bariumsulfat und Wasser für die Erhöhung der Kerbschlagzähigkeit (Izod, ermittelt gemäß ISO 180, wie in der Beschreibung beschrieben) eines Polymermaterials, das eine wiederkehrende Einheit der Formel 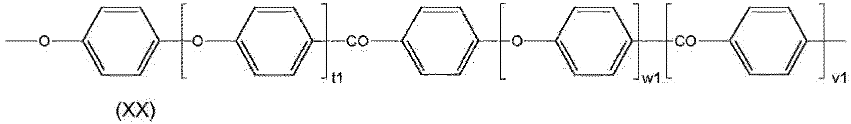 umfasst, worin t1 und w1 unabhängig voneinander 0 oder 1 bedeuten und v1 0, 1 oder 2 bedeutet, wobei der Quotient aus der in Gew.-% angegebenen Menge an Bariumsulfat und der in Gew.-% angegebenen Menge an Polymermaterial größer als 0,04 und kleiner als 0,4 ist; wobei die in Gew.-% angegebene Menge an Polymermaterials in einer Zusammensetzung, die das Bariumsulfat enthält, mindestens 50 Gew.-% beträgt.

2. Verwendung nach Anspruch 1, wobei c1=1,v1=0 und w1 = 0 ist.

3. Verwendung nach Anspruch 1 oder Anspruch 2, wobei der Quotient aus der in Gew.-% angegebenen Menge an Bariumsulfat und der in Gew.-% angegebenen Menge an Polymermaterial kleiner als 0,3 ist.

4. Verwendung nach einem der vorhergehenden Ansprüche, wobei die in Gew.-% angegebene Menge an Bariumsulfat in einer Zusammensetzung, die Bariumsulfat und das Polymermaterial enthält, mindestens 10 Gew.-% beträgt.

5. Verwendung nach einem der vorhergehenden Ansprüche, wobei das Bariumsulfat verwendet wird, um die Kerbschlagzähigkeit (Izod) des Polymermaterials um mindestens 10 KJm⁻² zu erhöhen.

6. Verwendung nach einem der vorhergehenden Ansprüche, wobei der Quotient aus der in Gew.-% angegebenen Menge an Wasser und Bariumsulfat im Bereich von 0,01 bis 0,2 liegt.

7. Verwendung nach einem der vorhergehenden Ansprüche, wobei die in Gew.-% angegebene Menge an Wasser in einer Zusammensetzung, die Bariumsulfat und das Polymermaterial enthält, kleiner als 1 Gew.-% ist.

8. Verfahren zur Erhöhung der Kerbschlagzähigkeit (Izod, ermittelt gemäß ISO 180, wie in der Beschreibung beschrieben) eines Polymermaterials, das eine wiederkehrende Einheit der Formel 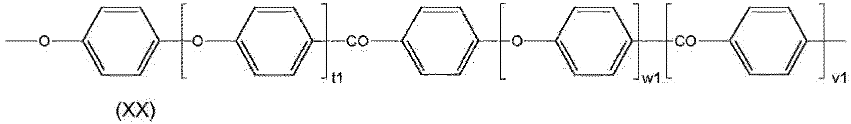 umfasst, worin t1 und w1 unabhängig voneinander 0 oder 1 bedeuten und v1 0, 1 oder 2 bedeutet, wobei das Verfahren das Erzeugen einer Zusammensetzung umfasst, die das Polymermaterial und Bariumsulfat umfasst, wobei der Quotient aus der in Gew.-% angegebenen Menge an Bariumsulfat und der in Gew.-% angegebenen Menge an Polymermaterial größer als 0,04 und kleiner als 0,4 ist; und wobei die in Gew.-% angegebene Menge an Polymermaterial in einer Zusammensetzung, die das Bariumsulfat enthält, mindestens 50 Gew.-% beträgt, wobei das Verfahren eine aktive Behandlung der Zusammensetzung umfasst, durch die der Wassergehalt, der in ihr enthalten ist, erhöht wird.

9. Verfahren nach Anspruch 8, wobei die in Gew.-% angegebene Menge an Wasser in dem Teil, das Bariumsulfat und das Polymermaterial enthält, mindestens 0,30 Gew.-% beträgt und weniger als 1 Gew.-% ist und die in Gew.-% angegebene Menge an Bariumsulfat in dem Teil mindestens 10 Gew.-% beträgt und kleiner als 30 Gew.-% ist.

10. Teil, das ein Polymermaterial umfasst, das eine wiederkehrende Einheit der Formel 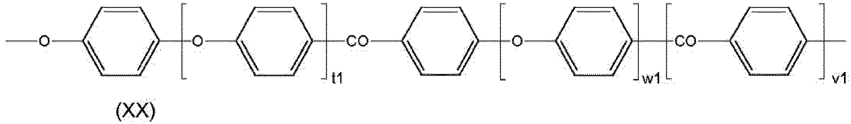 umfasst, worin t1 und w1 unabhängig voneinander 0 oder 1 bedeuten und v1 0, 1 oder 2 bedeutet, wobei der Quotient aus der in Gew.-% angegebenen Menge an Bariumsulfat und der in Gew.-% angegebenen Menge an Polymermaterial größer als 0,04 und kleiner als 0,4 ist; wobei die in Gew.-% angegebene Menge an Polymermaterial in einer Zusammensetzung, die das Bariumsulfat enthält, mindestens 50 Gew.-% beträgt; und wobei das Teil mindestens 0,1 Gew.-% Wasser, vorzugsweise mindestens 0,2 Gew.-%, noch bevorzugter mindestens 0,30 Gew.-% Wasser enthält.

11. Teil nach Anspruch 10, wobei das Teil in einer Verpackung bereitgestellt wird.

12. Teil nach Anspruch 11, wobei das Teil vakuumverpackt ist.

13. Teil nach Anspruch 10, wobei das Teil eine implantierbare Prothese ist.

## Revendications

1. Utilisation de sulfate de baryum et d'eau pour augmenter la résistance aux chocs Izod sur entaille (déterminée suivant la norme ISO 180 de la manière décrite dans la description) d'une matière polymère qui comprend un motif répété de formule 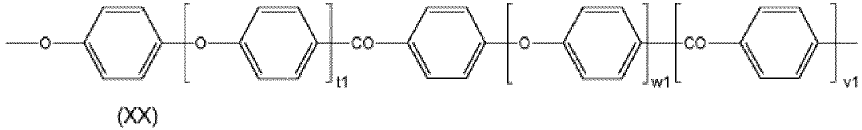 dans laquelle t1 et w1 sont égaux indépendamment à 0 ou 1 et v1 est égal à 0, 1 ou 2, le rapport du % en poids de sulfate de baryum au % en poids de matière polymère étant supérieur à 0,04 et étant inférieur à 0,4 ; le % en poids de ladite matière polymère dans une composition qui comprend ledit sulfate de baryum étant égal à au moins 50 % en poids.

2. Utilisation suivant la revendication 1, dans laquelle c1 = 1, v1 = 0 et w1 = 0.

3. Utilisation suivant la revendication 1 ou la revendication 2, dans laquelle le rapport du % en poids de sulfate de baryum au % en poids de ladite matière polymère est inférieur à 0,3.

4. Utilisation suivant l'une quelconque des revendications précédentes, dans laquelle le % en poids de sulfate de baryum dans une composition qui comprend du sulfate de baryum et ladite matière polymère est d'au moins 10 % en poids.

5. Utilisation suivant l'une quelconque des revendications précédentes, dans laquelle ledit sulfate de baryum est utilisé pour augmenter la résistance aux chocs Izod sur entaille de ladite matière polymère d'au moins 10 KJm⁻².

6. Utilisation suivant l'une quelconque des revendications précédentes, dans laquelle le rapport du % en poids d'eau du sulfate de baryum est dans l'intervalle de 0,01 à 0,2.

7. Utilisation suivant l'une quelconque des revendications précédentes, dans laquelle le % en poids d'eau dans une composition qui comprend du sulfate de baryum et ladite matière polymère est inférieure à 1 % en poids.

8. Procédé d'augmentation de la résistance aux chocs Izod sur entaille (déterminée suivant la norme ISO 180 de la manière décrite dans la description) d'une matière polymère qui comprend un motif répété de formule 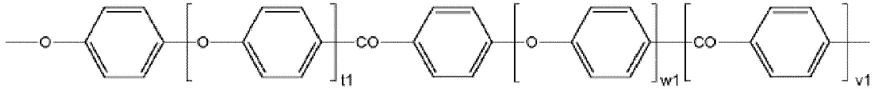 dans laquelle t1 et w1 sont égaux indépendamment à 0 ou 1 et v1 est égal à 0, 1 ou 2, le procédé comprenant la formation d'une composition comprenant ladite matière polymère et du sulfate de baryum, le rapport du % en poids de sulfate de baryum au % en poids de matière polymère étant supérieur à 0,04 et étant inférieur à 0,4 ; et le % en poids de ladite matière polymère dans une composition qui comprend ledit sulfate de baryum étant égal à au moins 50 % en poids, le procédé comprenant un traitement actif de ladite composition pour augmenter la quantité d'eau qu'elle renferme.

9. Procédé suivant la revendication 8, dans lequel le % en poids d'eau dans ladite pièce qui comprend du sulfate de baryum et ladite matière polymère est égal à au moins 0,30 % en poids et est inférieur à 1 % en poids ; le % en poids de sulfate de baryum dans ladite pièce est égal à au moins 10 % en poids et est inférieur à 30 % en poids.

10. Pièce qui comprend une matière polymère qui comprend un motif répété de formule 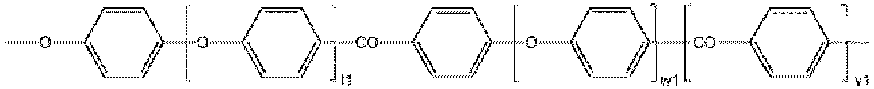 dans laquelle t1 et w1 sont égaux indépendamment à 0 ou 1 et v1 est égal à 0, 1 ou 2, le rapport du % en poids de sulfate de baryum au % en poids de matière polymère étant supérieur à 0,04 et étant inférieur à 0,4 ; le % en poids de ladite matière polymère dans une composition qui comprend ledit sulfate de baryum étant égal à au moins 50 % en poids ; et ladite pièce comprenant au moins 0,1 % en poids d'eau, avantageusement au moins 0,2 % en poids d'eau, plus avantageusement au moins 0,30 % en poids d'eau.

11. Pièce suivant la revendication 10, ladite pièce étant fournie dans un emballage.

12. Pièce suivant la revendication 11, ladite pièce étant emballée sous vide.

13. Pièce suivant la revendication 10, ladite pièce étant une prothèse implantable.
